# FASCICULE DE BREVET EUROPEEN

(11) **EP 1 875 882 B1**
(45) Date de publication et mention de la délivrance du brevet: **07.01.2015**
(21) Numéro de dépôt: 07354040.3
(22) Date de dépôt: 02.07.2007
(51) Int. Cl.: A61F 2/80

(54) **Dispositif d'étanchéité entre un manchon installé sur le moignon d'un membre amputé et une coque de prothèse**
Abdichtvorrichtung zwischen einer Halterung, die auf dem Stumpf eines amputierten Glieds angebracht ist, und einem Prothesenkörper
Watertight device between a sleeve installed on the stump of an amputated member and a prosthesis shell

(30) Priorité: 06.07.2006 FR 0606158
(43) Date de publication de la demande: 09.01.2008
(73) Titulaire: Chabloz, Pierre, 38450 Saint Georges de Commiers (FR)
(72) Inventeur: Chabloz, Pierre, 38450 Saint Georges de Commiers (FR)
(74) Mandataire: Hecké, Gérard

(56) Documents cités:
- EP-A1- 0 631 765
- WO-A-03/024370
- DE-U- 1 795 809
- US-A1- 2004 122 528

## Description

### Domaine technique de l'invention.

La présente invention est du domaine des prothèses non implantables dans le corps destinées à remplacer une partie manquante d'un membre amputé, tel qu'un bras ou une jambe par exemple. Elle a pour objet un dispositif d'étanchéité entre un manchon installé sur le moignon d'un membre amputé et une coque de prothèse, ainsi qu'une prothèse équipée d'un tel dispositif d'étanchéité.

### Etat de la technique.

Dans le domaine des prothèses, on connaît celles destinées à remplacer une partie manquante d'un membre amputé, tel qu'un bras ou une jambe par exemple. Une telle prothèse comporte un appendice terminal substitutif de la partie manquante du membre amputé, muni d'une coque d'emboîtement ménageant une cavité destinée à recevoir le moignon du membre amputé. La coque, notamment obtenue par moulage d'un matériau plastique, comporte un débouché pour l'introduction du moignon.

Il est recherché dans le domaine d'organiser la coque de manière à permettre une mise en place et un retrait du moignon qui soient simples et rapides, tout en garantissant une fiabilité de la jonction par emboîtement obtenue entre le moignon et la prothèse. A cet effet, il a été proposé d'organiser un emboîtement étanche à l'air entre le moignon et la coque pour interdire leur désolidarisation spontanée. Plus particulièrement, le moignon est préalablement enveloppé d'un manchon de forme extérieure complémentaire à la cavité de la coque, pour interdire un passage d'air entre eux et en conséquence retenir spontanément le moignon à l'intérieur de la coque. Pour autoriser la mise en place du moignon à l'intérieur de la coque, l'air résiduel contenu dans la cavité est chassé à travers un évent ménagé à la base de la coque. Cet évent est équipé d'un bouchon amovible prévu pour être mis en place après l'introduction du moignon à l'intérieur de la coque en vue d'interdire une introduction d'air à l'intérieur de la cavité, et prévu pour être retiré en vue d'autoriser une entrée d'air à l'intérieur de la cavité lorsque l'utilisateur souhaite un retrait de la prothèse.

Pour garantir l'étanchéité à l'air entre le manchon et la coque, un organe d'étanchéité est placé à l'extrémité proximale de la coque. Plus particulièrement, cet organe d'étanchéité est constitué d'un joint à lèvre qui est interposé entre la coque et le manchon en étant indifféremment fixé sur l'un de ceux-ci. De manière courante, ce joint est en polymère et est rapporté par collage à l'intérieur de la coque ou à l'extérieur du manchon.

De telles modalités d'organisation de l'étanchéité entre la coque et le manchon induisent une détérioration rapide et un risque d'arrachement du joint, en raison des manoeuvres répétées d'introduction et de retrait du manchon. Il en ressort une pérennité insuffisante et une fiabilité douteuse des moyens d'étanchéité interposés entre la coque et le manchon. C'est pourquoi il a été proposé dans le domaine d'agencer le joint en diaphragme annulaire dont le bord extérieur comporte un bourrelet destiné à être logé à l'intérieur d'une réserve ménagée sur la coque. Un jonc torique est noyé à l'intérieur du bourrelet, pour éviter une échappée du bord du joint hors de la réserve.

Un tel agencement du joint en diaphragme annulaire est décrit dans le document EP-A-631765. Cependant, l'organisation du bord du joint en bourrelet à l'intérieur duquel est noyé le jonc, rend délicate sa mise en place à l'intérieur de la réserve. Une telle mise en place aléatoire n'offre pas une garantie suffisante au regard d'un risque d'échappée du bord du joint hors de la réserve. Il est aussi opportun de réduire les coûts d'obtention du joint, pour finalement réduire les coûts de maintenance liés à son remplacement. Les modalités mises en oeuvre pour le retrait et la mise en place du joint méritent d'être améliorées, pour permettre à l'utilisateur de remplacer aisément le joint à moindre frais.

### Objet de l'invention.

Le but de la présente invention est de proposer un dispositif d'étanchéité entre un manchon installé sur le moignon d'un membre amputé et une coque de prothèse, qui soit pérenne et fiable sans néanmoins induire des coûts d'obtention et de remplacement importants, qui ne provoque aucune altération de la coque et/ou du manchon, et qui permet une maintenance rapide et aisée.

Le dispositif de la présente invention est un dispositif d'étanchéité entre une coque d'une prothèse recevant un manchon installé sur le moignon d'un membre amputé. La coque délimite une cavité de réception du manchon. Un joint déformable est interposé entre la coque et le manchon pour interdire un passage d'air entre eux. Ce joint est conformé en diaphragme annulaire dont l'un des bords indifféremment intérieur et/ou extérieur est équipé d'un organe d'ancrage constitué d'un jonc torique. Ce jonc est destiné à être logé dans une réserve ménagée en correspondance dans le manchon et/ou dans la coque.

Selon la présente invention, un dispositif d'étanchéité pour prothèse du genre susvisé est principalement reconnaissable en ce que ledit bord du joint comporte un retour délimitant une capacité ouverte de réception du jonc, de sorte que le jonc soit amovible en vue de permettre sa mise en place et inversement son retrait aisés, et de sorte que le joint soit immobilisé par pincement de son bord correspondant entre le jonc et le fond de la réserve.

Le retour ménage un espace ouvert à l'intérieur duquel est logé le joint, et est placé en interposition entre le jonc et la paroi correspondante de la réserve. Une telle interposition permet la prise par pincement du bord du joint par l'organe d'ancrage, qui se trouve logé à l'intérieur de la réserve. Une telle prise par pincement permet une liaison fiable entre le joint et l'organe qui le reçoit, coque ou manchon, tout en autorisant un retrait et une mise en place aisés du jonc.

Le jonc pouvant être facilement introduit à l'intérieur et retiré hors de la capacité ouverte formée par le retour, le joint constitué seulement du diaphragme peut être aisément remplacé à moindre coût en conservant le jonc. Le coût d'obtention du joint est réduit, et sa mise en place est aisée à partir de l'introduction de son bord à l'intérieur de la réserve, et de la mise en place du jonc. Inversement le retrait volontaire du joint est aisé, en chassant le jonc et le bord du joint hors de la réserve. Pour remplacer le joint seul un remplacement du diaphragme suffit, le jonc étant susceptible d'être réutilisé. Grâce à la liberté relative entre le jonc et le diaphragme, le jonc est susceptible d'être soit formé d'un anneau fermé monobloc, soit d'être formé à partir d'un câble découpé à la longueur voulue puis mis en conformation annulaire.

La réserve est de préférence ménagée dans la paroi de la coque, mais est aussi susceptible d'être ménagée dans la paroi du manchon.

La réserve est avantageusement formée de moulage simultanément à la formation par moulage de la coque. La réserve est notamment formée à partir d'un noyau agencé en gabarit qui est introduit à l'intérieur du moule.

### Description des figures.

La présente invention sera mieux comprise, et des détails en relevant apparaîtront, à la lecture de la description qui va être faite d'une forme préférée de réalisation en relation avec les figures de la planche annexée, dans laquelle :
La fig.1 est une représentation schématique en coupe axiale d'un dispositif de jonction par emboîtement étanche entre une coque de prothèse et un manchon prévu pour être installé sur le moignon d'un membre amputé.
La fig.2 est une représentation schématique en coupe axiale d'une coque d'un dispositif de jonction illustré sur la fig.1.
La fig.3 est un détail de la fig.2, illustrant les modalités d'ancrage d'un joint d'étanchéité sur la coque.

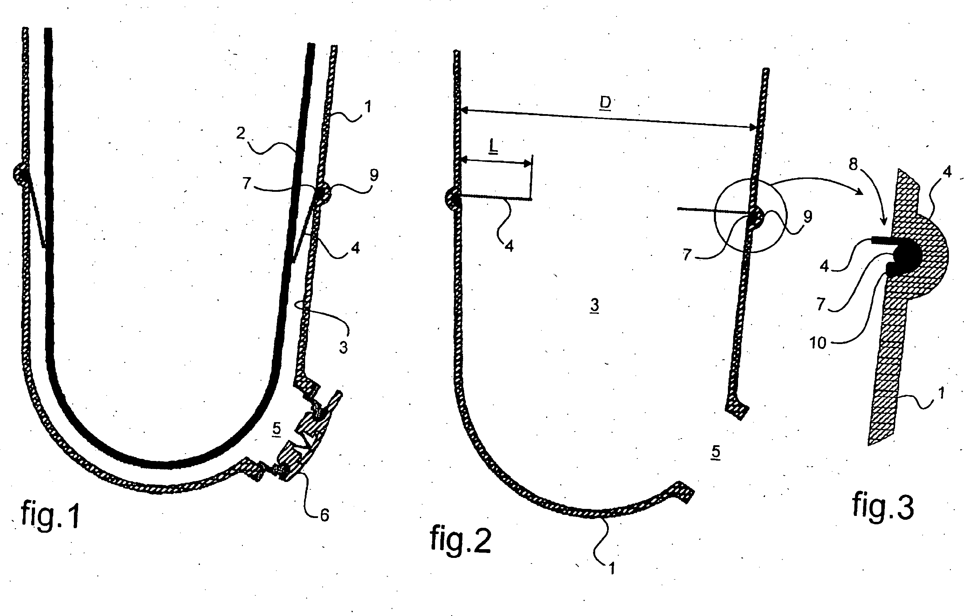

Sur la fig.1, une coque 1 de prothèse est destinée à recevoir par emboîtement étanche un manchon 2 installé sur le moignon d'un membre amputé. La conformation extérieure du manchon 2 est de forme complémentaire à celle d'une cavité 3 que comporte la coque 1 pour recevoir le manchon 2. Pour retenir la coque 1 sur le manchon 2, des moyens de type à dépression sont mis en oeuvre. Plus particulièrement, une étanchéité est organisée entre la coque 1 et le manchon 2, de sorte que le manchon 2 étant introduit à l'intérieur de la coque 1, un passage d'air vers la cavité 3 soit interdit. Cette interdiction met en oeuvre un joint d'étanchéité qui est placé à l'extrémité proximale de la coque 1, en interposition entre celle-ci et le manchon 2. L'introduction du manchon 2 à l'intérieur de la cavité 3 est autorisée malgré la présence du joint 4, grâce à un évent 5 qui est ménagé à la base de la coque 1. Cet évent 5 est muni d'un bouchon 6, qui intègre avantageusement de moulage une valve.

Sur les fig.2 à fig.3, le joint 4 est constitué d'un diaphragme conformé en anneau de faible épaisseur et d'une largeur L conséquente. A titre indicatif, cette largeur L est comprise entre 15% et 35% du diamètre D de l'anneau. La conformation du joint 4 en anneau lui confère une souplesse importante favorisant sa déformabilité. Le joint 4 est fixé à la coque 1 par l'intermédiaire d'un organe d'ancrage conformé en jonc 7 torique, qui est en prise sur l'un des bords 8 de l'anneau, bord extérieur plus particulièrement. Ce jonc 7 est élastiquement déformable, et est destiné à être logé dans une réserve 9 périphérique que comporte la coque 1. Cette réserve 9 est notamment formée par moulage conjointement à la formation de la coque 1. L'élasticité du jonc permet son introduction aisée dans la réserve 9, à l'intérieur de laquelle il est spontanément maintenu à partir de son expansion radiale naturelle.

Le bord 8 du joint 4 comporte un retour 10 qui délimite une capacité ouverte de réception de l'organe d'ancrage 7. Cette capacité est ouverte pour permettre aisément une mise en place et un retrait du jonc 7, indépendamment d'une mise en place et d'un retrait du joint 4. Le bord 8 du joint 4 est comprimé entre l'organe d'ancrage 7 et le fond de la réserve 9, garantissant une fixation fiable du joint 4 sur la coque 1. L'association entre la conformation annulaire du joint 4 et ses modalités de fixation par l'intermédiaire d'un jonc 7 élastiquement déformable susceptible d'être séparé du joint 4, confèrent une réversibilité aisée de la fixation du joint 4 et garantissent la fiabilité de l'étanchéité obtenue entre la coque 1 et le manchon 2, grâce à la déformabilité de l'anneau dont la qualité d'ancrage n'est pas altérée par sa souplesse. Un remplacement du joint 4 peut être rapidement et aisément effectué à partir d'un retrait du jonc 7 hors de la cavité formée par le retour ménagé en bordure du joint 4, pour rompre le pincement de ce dernier entre le jonc 7 et le fond de la réserve 9.

## Revendications

1. Dispositif d'étanchéité entre une coque (1) d'une prothèse recevant un manchon (2) adapté à être installé sur le moignon d'un membre amputé, la coque (1) délimitant une cavité (3) de réception du manchon (2), ledit dispositif comprenant un joint (4) déformable étant interposé entre la coque (1) et le manchon (2) pour interdire un passage d'air entre eux, ce joint (4) étant conformé en diaphragme annulaire dont l'un des bords (8) indifféremment intérieur et/ou extérieur est équipé d'un organe d'ancrage constitué d'un jonc (7) torique destiné à être logé dans une réserve (9) ménagée en correspondance dans le manchon (2) et/ou dans la coque (1), **caractérisé en ce que** ledit bord (8) du joint (4) comporte un retour (10) délimitant une capacité ouverte de réception du jonc (7), de sorte que le jonc (7) est amovible en vue de permettre sa mise en place et inversement son retrait aisés et de sorte que le joint (4) est immobilisé par pincement de son bord (8) correspondant entre le jonc (7) et le fond de la réserve (9).

2. Dispositif d'étanchéité selon la revendication 1, **caractérisé en ce que** la réserve (9) est ménagée dans la paroi de la coque (1).

3. Dispositif d'étanchéité selon la revendication 1, **caractérisé en ce que** la réserve (9) est ménagée dans la paroi du manchon (2).

4. Dispositif d'étanchéité selon la revendication 2, **caractérisée en ce que** la réserve (9) est formée de moulage simultanément à la formation par moulage de la coque (1).

## Patentansprüche

1. Abdichtvorrichtung zwischen einem Körper (1) einer Prothese, die eine Halterung (2) aufnimmt, die geeignet ist, auf dem Stumpf eines amputierten Glieds angebracht zu werden, wobei der Körper (1) eine Kavität (3) zur Aufnahme der Halterung (2) begrenzt, welche Vorrichtung eine verformbare Dichtung (4) umfasst, die zwischen dem Körper (1) und der Halterung (2) vorgesehen ist, um einen Luftdurchzug zwischen ihnen zu verhindern, wobei diese Dichtung (4) in Form einer ringförmigen Membran vorgesehen ist, deren einer Rand (8) - der innere und/oder der äußerte - mit einem von einem O-Ring (7) gebildeten Verankerungselement versehen ist, das dazu bestimmt ist, in einem Raum (9) untergebracht zu werden, der entsprechend in der Halterung (2) und/oder in dem Körper (1) vorgesehen ist, **dadurch gekennzeichnet, dass** der Rand (8) der Dichtung (4) einen Rücksprung (10) aufweist, der einen offenen Aufnahmeraum für den O-Ring (7) begrenzt, sodass der O-Ring (7) im Hinblick darauf abnehmbar ist, dass er problemlos eingesetzt und umgekehrt herausgenommen werden kann, und sodass die Dichtung (4) festgesetzt ist durch Einklemmen ihres entsprechenden Randes (8) zwischen dem O-Ring (7) und dem Boden des Raums (9).

2. Dichtungsvorrichtung nach Anspruch 1, **dadurch gekennzeichnet, dass** der Raum (9) in der Wand des Körpers (1) vorgesehen ist.

3. Dichtungsvorrichtung nach Anspruch 1, **dadurch gekennzeichnet, dass** der Raum (9) in der Wand der Halterung (2) vorgesehen ist.

4. Dichtungsvorrichtung nach Anspruch 2, **dadurch gekennzeichnet, dass** der Raum (9) gleichzeitig mit dem Formgießen des Körpers (1) formgegossen wird.

## Claims

1. Seal device between a shell (1) of a prosthesis receiving a sleeve (2) suited for being installed on the stump of an amputated member, the shell (1) confining a cavity (3) for receiving the sleeve (2), said device comprising a ductile gasket(4) inserted between the shell (1) and the sleeve (2) to prevent air from passing between them, said gasket (4) being shaped in the form of an annular diaphragm one edge (8) of which - equally the inner and/or the outer one - is provided with an anchoring means composed of an O-ring (7) intended to be housed in a gap (9) provided correspondingly in the sleeve (2) and/or the shell (1), **characterized in that** said edge (8) of the gasket (4) comprises a return (10) confining an open volume for reception of the O-ring (7) so that the O-ring (7) is removable for permitting its easy insertion and conversely its easy withdrawal and so that the gasket (4) is immobilized because of the pinching of its corresponding edge (8) between the O-ring (7) and the bottom of the gap (9).

2. Seal device according to claim 1, **characterized in that** the gap (9) is provided in the wall of the shell (1).

3. Seal device according to claim 1, **characterized in that** the gap (9) is provided in the wall of the sleeve (2).

4. Seal device according to claim 2, **characterized in that** the gap (9) is molded at the same time as molding of the shell (rut) takes place (1).
